# FASCICULE DE BREVET EUROPEEN

(11) **EP 4 358 985 B1**
(45) Date de publication et mention de la délivrance du brevet: **28.01.2026**
(21) Numéro de dépôt: 22740945.5
(22) Date de dépôt: 17.06.2022
(51) Int. Cl.: A61K 36/30, A61Q 5/12, A61K 8/9789, A61Q 1/10, A61Q 19/00, A61Q 3/00, A61Q 5/00, A61Q 5/02, A61Q 7/00

(54) **UTILISATION COSMETIQUE D'UN EXTRAIT DE PHACELIE, NOTAMMENT POUR LE SOIN DES PHANERES**
KOSMETISCHE VERWENDUNG EINES PHYLLIA-EXTRAKTS, INSBESONDERE ZUR PFLEGE VON HAUTANHANGSGEBILDEN
COSMETIC USE OF A PHACELIA EXTRACT, IN PARTICULAR FOR THE CARE OF SKIN APPENDAGES

(30) Priorité: 21.06.2021 FR 2106585
(43) Date de publication de la demande: 01.05.2024
(73) Titulaire: Laboratoires De Biologie Vegetale Yves Rocher, 56200 La Gacilly (FR)
(72) Inventeur: TOUZEAU, Betty, 92370 CHAVILLE (FR); ZOZINE, Sophie, 91630 MAROLLES EN HUREPOIX (FR); LAPERDRIX, Céline, 78470 SAINT-REMY-LES-CHEVREUSE (FR)
(74) Mandataire: Novagraaf Technologies
(86) Numéro de dépôt international: PCT/FR2022/051182
(87) Numéro de publication internationale: WO 2022/269176

(56) Documents cités:
- WO-A1-2012/129683
- CN-A- 112 569 134
- US-A- 5 824 312
- US-A1- 2011 217 753
- BAJKACZ SYLWIA ET AL: "Determination of Flavonoids and Phenolic Acids in Plant Materials Using SLE-SPE-UHPLC-MS/MS Method", FOOD ANALYTICAL METHODS, SPRINGER NEW YORK LLC, US, vol. 11, no. 12, 6 August 2018 (2018-08-06), pages 3563 - 3575, XP036621751, ISSN: 1936-9751, [retrieved on 20180806], DOI: 10.1007/S12161-018-1332-9
- ANONYMOUS: "Native Plant Fun Facts: Phenomenal Phacelia! - Garden for the Environment", 30 March 2020 (2020-03-30), XP055893519, Retrieved from the Internet <URL:https://www.gardenfortheenvironment.org/growing-gardeners-archive/2020/3/30/native-plant-fun-facts-2-phenomenal-phacelia> [retrieved on 20220218]
- ANONYMOUS: "Phacelia", 13 April 2021 (2021-04-13), XP055893537, Retrieved from the Internet <URL:https://web.archive.org/web/20210413235409/http://www.worldbotanical.com/phacelia.htm> [retrieved on 20220218]

## Description

### Domaine technique de l'invention

La présente invention se rapporte à une utilisation cosmétique non thérapeutique d'au moins un extrait hydrophile de phacélie appartenant à l'espèce *Phacelia tanacetifolia,* choisi parmi un extrait de feuille, de tige, de fleur, de graine, ou l'un de leurs mélanges pour le soin, le nettoyage et/ou le maquillage des phanères.

La présente invention trouve une application dans le domaine de la cosmétique.

### Etat de la technique

A l'instar de la peau, les cheveux et les ongles accumulent les dégâts engendrés par les stress environnementaux comme les UVs, la pollution, le froid, mais également les traitements esthétiques comme le séchage, le frisage, le lissage, la décoloration, la coloration et le vernissage, ou encore l'utilisation de produits agressifs, comme des dissolvants pour les ongles ou des shampoings trop récurrents ou mal adaptés pour les cheveux. De plus, ils sont affectés par des facteurs internes comme le stress, les modifications hormonales ou encore le type d'alimentation.

Le cheveu est constitué, dans sa partie visible, de 3 couches:
- la cuticule, d'une épaisseur de 3,5 à 4,5 µm, est la couche la plus externe. Elle est constituée de plaques cornées en forme d'écailles qui se recouvrent partiellement et constituent une protection imperméable pour la tige du cheveu,
- vient ensuite le cortex, représentant 80% du cheveu. Il est composé principalement de protéines (kératine essentiellement) et confère à la fibre résistance et solidité de par sa haute organisation. Le cortex contient en outre les granules de mélanines, donnant au cheveu sa couleur,
- enfin la moelle, composée de cellules mortes, qui a une texture molle et graisseuse.

La plaque de l'ongle, fabriquée par la matrice unguéale, est formée de kératine peu hydratée, avec seulement 3 % d'eau, et contient très peu de lipides (0,15 à 0,75 %). Elle est, tout comme la peau, recouverte par un film hydrolipidique, qui assure le maintien de l'hydratation et la protection contre les bactéries.

La kératine constitue donc le composant principal des cheveux, et par extension des cils et des sourcils, et des ongles, et assure leur protection et leur imperméabilité. Cette protéine est synthétisée par les kératinocytes, produits dans la matrice unguéale ou la matrice du bulbe pileux.

La croissance des ongles a ainsi lieu au niveau de la matrice unguéale où se renouvellent les kératinocytes. Ces derniers vont, au fur et à mesure, se différencier et traverser les différentes couches pour atteindre la surface. Lors de leur ascension, les kératinocytes créent une grande quantité de kératine « dure ». Ces cellules restent telles quelles et sont pouorées par la prochaine arrivée de kératinocytes, ce qui permet aux ongles de pousser de façon permanente (environ 1mm par semaine) et de se renouveler complètement en 3 à 6 mois (9 à 12 mois pour les ongles des pieds). Toutefois, la pousse ralentit avec l'âge et la qualité de la matrice et des kératines produites est moindre.

La croissance des cheveux a lieu, quant à elle, dans la matrice du bulbe pileux, où les kératinocytes se divisent en cellules filles pendant toute la phase anagène du cycle de vie d'un cheveu. À peine formées, les cellules filles se dédoublent aussitôt, évincent les précédentes, leur font perdre leur noyau, les font mourir et se rigidifier, en les repoussant vers le haut. Puis elles meurent à leur tour pour laisser place aux suivantes. La kératine naît de ce stockage de cellules mortes (différenciées) qui durcissent (kératinisation) et remontent le long du follicule pileux, pour former la surface du cheveu.

La croissance des ongles comme des cheveux est fortement impactée par les anses capillaires fortement présentes à la base des phanères pour apporter nutriments et eau nécessaires aux bons fonctionnements cellulaires.

La croissance des ongles et des cheveux, ainsi que leur résistance, dépendent donc directement des capacités prolifératives des kératinocytes. Or, les stress internes (hormones, hygiene de vie, traitements médicaux...), extérieurs (UV, pollution, détergents...) et l'âge altèrent les kératinocytes et ralentissent ce processus, qui peut alors devenir plus long et plus irrégulier. Les cheveux et les ongles deviennent ainsi moins résistants, cassants, ternes, et présenter une croissance ralentie.

Le marché des produits cosmétiques est très important et les attentes des consommateurs/consommatrices sont très élevées. En termes de soin des cheveux, des ongles, mais aussi des cils et des sourcils, ils/elles s'orientent de plus en plus vers des produits efficaces combinant également naturalité et écoconception.

Il existe ainsi un réel besoin de trouver de nouvelles compositions et/ou composés d'origine naturelle, permettant d'améliorer l'aspect et les qualités de surface des cheveux, des ongles, des cils et des sourcils, ainsi que leur croissance.

L'utilisation traditionnelle de d'extraits de phacélie (Phacelia californica) pour traiter des affections cutanées (jus de feuilles fraîches pour traiter des maladies de la peau) ainsi que l'utilisation d'extraits aqueux de racines de phacélie sous forme de thé pour traiter la fièvre et les rhumes est décrite dans "Native Plant Fun Facts: Phenomenal Phacelia!-Garden for the Environment", https://www.gardenfortheenvironment.org/growing-gardeners-archive/2020/3/30/native-plant-fun-facts-2-phenomenal-phacelia (2020-03-30). Des espèces de phacélie et des utilisations traditionnelles de leurs extraits dont l'utilisation des racines de P. californica pour la fièvre, le rhume et la toux et l'utilisation des feuilles en dermatologie sont également décrites dans "Phacelia", https://web.archive.org/web/20210413235409/http:// www.worldbotanical.com/phacelia.htm (13.04.2021).

### Description de l'invention

La présente invention a précisément pour but de répondre à ces besoins et inconvénients de l'art antérieur.

Les inventeurs sont les tout premiers à utiliser un extrait de phacélie, permettant précisément de répondre efficacement aux besoins précités.

Les inventeurs de la présente ont mis en évidence des propriétés de l'extrait qui n'avaient jamais été décrites ou suggérées dans l'art antérieur. En effet, de manière surprenante, les inventeurs ont mis en évidence que l'extrait de phacélie possède une action cosmétique, notamment adaptée au soin des phanères.

Les inventeurs ont notamment mis en évidence un effet de l'association des extraits de phacélie sur l'esthétisme en général des cheveux, des ongles, des cils et des sourcils. Ils ont démontré que l'extrait de phacélie améliore les processus de différenciation kératinocytaire et promeut donc la kératinisation et la croissance des fibres capillaires, et des phanères en général.

Ainsi, un premier objet de l'invention se rapporte à une utilisation cosmétique non thérapeutique d'au moins un extrait hydrophile de phacélie appartenant à l'espèce *Phacelia tanacetifolia,* choisi parmi un extrait de feuille, de tige, de fleur, de graine, ou l'un de leurs mélanges pour le soin, le nettoyage et/ou le maquillage des phanères.

On entend par « phacélie », au sens de la présente invention, l'espèce Phacelia tanacetifolia.

Selon l'invention, l'extrait de phacélie est un extrait de feuille, ou un extrait de tige, ou un extrait de fleur, ou encore un mélange d'extrait de feuilles, de tiges et/ou de fleurs. S'il s'agit d'un extrait des parties aériennes fleuries, celles-ci peuvent être récoltées en début de floraison (bourgeon), en pleine floraison ou en fin de floraison.

L'extrait hydrophile de phacélie peut être comme défini dans la revendication 3II peut s'agir notamment d'un extrait choisi parmi un extrait alcoolique, par exemple un extrait éthanolique, un extrait aqueux, par exemple un hydrodistillat, comme une eau florale, un extrait hydro-alcoolique, par exemple séché ou liquide avec reprise ou non dans un solvant cosmétique, ou un extrait glycolique.

L'extrait de phacélie peut être obtenu par tout procédé d'extraction connu de l'homme du métier pour d'autres plantes et adapté à la phacélie. Ainsi, le solvant d'extraction peut être choisi parmi l'eau, les alcools en C1 à C5, par exemple l'éthanol, le méthylpropanediol, le propane-1,3-diol; des glycols en C3 à C5, par exemple le propylène glycol, le butylène glycol, dipropylène glycol; le pentylène glycol, la glycérine l'hexane, l'heptane, l'acétate d'éthyle, le CO2, les lipides et huiles végétales ; ou leurs mélanges. Par exemple, le solvant d'extraction peut être l'éthanol, avec une dilution dans un glycol comme la glycérine, le pentylène glycol, le propylène glycol ou le propanediol, ou dans une huile comme l'huile de jojoba, de macadamia et/ou de tournesol.

De façon avantageuse, le solvant d'extraction employé peut être un solvant hydroéthanolique, de préférence dans un mélange eau/éthanol ayant un rapport massique eau/alcool compris entre 90/10 et 10/90, par exemple de l'ordre de 70/30. L'extrait de phacélie peut être obtenu, à pression atmosphérique, par macération, infusion, décoction, effleurage, lixiviation, ultra-sons ou micro-ondes, ou sous haute pression avec des solvants à l'état super ou subcritque comme le CO2 ou l'eau, avec ou sans co-solvant, par entrainement à la vapeur d'eau (hydrodistillation). Par exemple, l'extrait de phacélie peut être obtenu en broyant (ou non) une ou plusieurs parties de la plante, fraiches, congelées, de préférence séchées et coupées ou broyées au préalable, puis en mettant en contact le broyat avec un solvant tel que défini précédemment, préférentiellement un mélange eau/alcool ayant un rapport eau/alcool entre 90/10 et 10/90, par exemple un mélange eau/alcool ayant un rapport massique 70/30. L'extraction peut être conduite en mélangeant le broyat et le solvant et en soumettant ce mélange à une agitation de façon à optimiser les échanges entre le broyat et le solvant d'extraction. L'extraction peut être conduite à une température de 20 à 100°C. Par exemple, l'extraction peut être réalisée pendant une durée de l'ordre de 1/2 à 4 heures. La quantité de plante utilisée par rapport au solvant peut être par exemple de 0,5 à 20 % en poids par rapport au poids total de plantes et de solvant, par exemple 1,0 à 15,0%, ou 3,0 à 12,0% ou 6,0 à 8,0 %, par exemple environ 7%. Il est à noter que l'extrait employé dans la présente invention peut éventuellement être purifié préalablement à sa mise en œuvre dans une composition cosmétique. Cette purification peut par exemple être effectuée par extraction liquide-liquide, par précipitation ou par chromatographie préparative. L'extrait employé dans la présente invention peut être dilué dans un solvant connu de l'homme du métier, comme par exemple le propylène glycol, le butylène glycol, le dipropylène glycol, le pentylène glycol, le méthylpropanediol, le propane-1,3-diol, la glycérine, l'eau ou un mélange de ces solvants. Il peut s'agir par exemple d'un extrait obtenu à partir de parties aériennes fleuries, notamment extraites par un mélange hydroéthanolique et dilué dans la glycérine.

Selon un mode de réalisation, on utilise comme extrait de phacélie, l'extrait brut obtenu qui est issu de l'extraction décrite ci-dessus. Il peut donc s'agir du milieu tel qu'il est obtenu directement à l'issue de l'extraction par un des solvants précités, cet extrait brut étant éventuellement filtré préalablement avant son emploi dans une composition cosmétique. Dans ce mode de réalisation, l'extrait employé se présente sous la forme d'une dispersion ou d'une solution dans un milieu liquide incluant le solvant précité.

Selon un autre mode de réalisation, on utilise un extrait séché obtenu en soumettant l'extrait brut précité, de préférence filtré, à une étape ultérieure de séchage, typiquement par atomisation ou par lyophilisation, selon des techniques connues, permettant de préserver l'intégrité des composés présents dans l'extrait. L'extrait obtenu selon ce mode de mise en œuvre se présente généralement sous la forme d'une poudre qui peut être employée telle qu'elle ou reprise dans un solvant ou un dispersant pour formuler une composition cosmétique.

Un extrait utilisé dans l'invention est susceptible d'être préparé selon le procédé comprenant les étapes suivantes :
a) Mise en contact de tout ou partie de la plante avec un solvant de type hydroalcoolique, préférentiellement à raison d'au moins 50g/L,
b) Extraction,
c) Séparation des phases soluble et insoluble
d) Filtrations de l'extrait brut jusqu'à la filtration stérilisante
e) Eventuellement concentration du produit obtenu à l'étape d),
f) Eventuellement, dilution dans un solvant approprié ou ajout de toute substance permettant de finaliser l'extrait,
g) Eventuellement, pasteurisation et/ou filtration stérilisante de l'extrait obtenu dans les étapes antérieures.

Des étapes de décoloration et désodorisation de la phase soluble peuvent être envisagées sans modifier la fraction active.

On entend par « composition cosmétique », dans la présente invention, toute composition à visée cosmétique, c'est à dire esthétique, pouvant être mise en contact avec les parties superficielles du corps humain, par exemple l'épiderme, les systèmes pileux et capillaires, les organes externes, les dents, les ongles et les muqueuses externes. Avantageusement, une composition cosmétique permet, exclusivement ou principalement, de les protéger, parfumer, maintenir en bon état, modifier leur aspect ou en corriger les défauts superficiels. Notamment, une composition cosmétique peut comprendre un véhicule cosmétiquement acceptable.

Par « véhicule cosmétiquement acceptable », on entend un véhicule adapté pour une utilisation en contact avec les fibres kératiniques, en particulier celles des cheveux, des ongles, des cils et des sourcils, sans toxicité, irritation, réponse allergique indue et similaire. Le véhicule cosmétiquement acceptable peut être choisi parmi l'eau, l'allantoïne, la glycérine, le méthylpropanediol, les tensio-actifs, cette liste n'étant pas limitative.

La composition cosmétique utilisée dans l'invention peut être obtenue par tout procédé approprié connu de l'homme du métier pour la fabrication d'une composition cosmétique. Il peut s'agir, par exemple d'un simple mélange. Il peut s'agir également, par exemple, d'un procédé comprenant une étape d'incorporation d'une phase interne dans une phase externe au moyen d'un émulseur, par exemple d'une turbine de type rotor-stator. Il peut s'agir également par exemple d'un procédé utilisant la Température d'Inversion de Phase (TIP), ce procédé étant classiquement utilisé par l'homme de l'art pour obtenir des émulsions huile dans eau dont les gouttelettes dispersées sont particulièrement fines, par exemple avec un diamètre de 0,1 à 1 µm.

Dans la composition utilisée dans l'invention, la teneur en extrait de phacélie peut être compris entre 0,01 et 10% du poids total de la composition. Par exemple, la composition peut comprendre de 0,01 à 5 %, ou de 0,01 à 3 %, ou de 0,01 à 2 %, ou de 0,01 à 1 %, ou de 0,1 à 3 %, ou de 0,1 à 1%, ou de 0,05 à 5%, en poids de l'extrait de phacélie par rapport au poids total de la composition.

La composition cosmétique utilisée dans l'invention peut se trouver sous toute forme appropriée pour une application cosmétique ou dermatologique. Avantageusement, la composition est une composition à usage topique, rincée ou non rincée.

Il peut s'agir par exemple d'une composition sous une forme choisie dans le groupe comprenant une émulsion huile dans eau, ou eau dans huile, ou un mélange de ces émulsions.

La composition peut notamment être sous une forme choisie parmi un shampooing liquide ou solide, un après-shampooing liquide ou solide, un produit coiffant tel qu'une lotion de mise en plis, une lotion pour le brushing, ou une composition de fixation ou de coiffage telle qu'une laque ou un spray, un vernis à ongles, une base de soin pour les ongles, un mascara, une solution, un gel, un baume, un beurre, une lotion, une suspension, une mousse, un savon, un onguent, une crème, une huile et une émulsion, cette forme pouvant être rincée ou non rincée.

La composition cosmétique peut par exemple se trouver sous une forme choisie dans le groupe comprenant un gel aqueux ou hydroalcoolique, une crème aqueuse ou hydroalcoolique et une lotion aqueuse ou hydroalcoolique. Ces formulations utilisables pour la mise en œuvre de la présente invention sont connues dans l'état de la technique par les formulateurs. Dans ces exemples de composition, il suffit d'ajouter l'association des huiles essentielles pour obtenir une composition utilisable dans la présente invention.

L'extrait utilisé dans la présente invention peut être utilisé dans une composition cosmétique seul ou en combinaison avec une ou plusieurs autres substances ou ingrédients actifs ou inactifs cosmétiquement ou dermatologiquement. Avantageusement, la composition utilisée dans l'invention peut notamment comprendre un ou plusieurs agents cosmétiques connus de l'homme du métier, autres que les extraits mentionnés dans l'invention. Il peut s'agir notamment de tout extrait ou molécule connu pour stiumler la pousse des cheveux et/ou la différenciation des kératinocytes, comme par exemple les inulines, par exemple celles citées dans le document EP3592434, les acides aminés comme l'arginine, la cystéine, la méthionine, la cystine, la tyrosine, des protéines végétales comme par exemple les protéines de lupin blanc, des mucilages, ou encore des huiles végétales comme l'huile de jojoba ou l'huile de ricin.

Les substances ou ingrédients inactifs sont ceux qui n'agissent pas cosmétiquement ou dermatologiquement. Il s'agit des éléments de la composition permettant notamment d'accompagner l'extrait, de constituer une formulation particulière, de conserver l'extrait actif dans le temps, etc. Il peut s'agir en outre de tout produit de base pouvant se trouver dans les compositions cosmétiques ou dermatologiques classiques. Par opposition, les substances ou ingrédients actifs sont ceux qui dans l'application cosmétique ou dermatologique visée ont une action esthétique et/ou médicale.

Un procédé de préparation d'un extrait de phacélie choisi parmi un extrait de feuille, de tige, de fleur, de graine, de racine, ou l'un de leurs mélanges est décrit, le procédé comprenant une étape d'extraction choisie parmi une extraction alcoolique, aqueuse, hydro-alcoolique, glycolique, une macération huileuse et une extraction lipidique. Ces étapes peuvent comprendre les caractéristiques techniques telles que décrit ci-avant.

Un extrait de phacélie choisi parmi un extrait alcoolique, un extrait aqueux, par exemple un hydrodistillat, comme une eau florale, un extrait hydro-alcoolique, par exemple séché ou liquide avec reprise ou non dans un solvant cosmétique, et un extrait glycolique, un macérat huileux, et un extrait lipidique, pouvant comprendre par exemple une extraction CO2 supercritique, est décrit. Par exemple, l'extrait utilisé dans l'invention est susceptible d'être obtenu par un procédé de préparation tel que définie ci-avant.

Un dispositif se présentant sous une forme choisie parmi un pot, un flacon, un flacon-pompe, un vaporisateur, un récipient aérosol, un spray, une capsule ou une gélule, le dispositif comprenant une composition ou un extrait de phacélie tels que défini ci-avant, est décrit.

L'utilisation cosmétique d'un extrait de phacélie, ou d'une composition cosmétique telle que définie ci-avant pour le soin, le nettoyage et/ou le maquillage des phanères, permet avantageusement de stimuler l'angiogenèse au niveau des zones des bulbes et des matrices ungueales, et ainsi d'assurer une bonne croissance des ongles et des cheveux/cils/sourcils.

On entend par « phanères », au sens de la présente invention, au moins un élément choisi parmi les ongles, les cheveux, les cils, les poils et les sourcils.

L'action cosmétique vise notamment à améliorer l'aspect de surface, et/ou la croissance des phanères. On entend par « améliorer l'aspect de surface » tout effet permettant d'embellir, notamment rendre plus brillant, rendre plus doux, rendre plus lisse, rendre moins cassants, donner un aspect plus épais, et/ou renforcer les ongles ou cheveux mous. Il s'agit donc d'une action purement esthétique, à l'exclusion de toute action thérapeutique.

Avantageusement, l'action esthétique sus-mentionnée peut être due à une amélioration de la kératinisation des phanères. Cette amélioration de la kératinisation des phanères peut avantageusement être due à l'action de l'extrait de phacélie sur les mécanismes de différenciation kératinocytaire.

Un procédé de soin cosmétique non-thérapeutique, comprenant l'application sur les phanères d'une composition cosmétique, ou d'un extrait de phacélie tels que définis ci-avant, est décrit. Avantageusement, le procédé permet de stimuler la kératinisation des phanères chez un humain ou un animal. Cette stimulation peut permettre, de manière avantageuse, au moins une action sur les phanères choisie parmi protéger, embellir, rendre plus brillant, rendre plus doux, rendre plus lisse, rendre moins cassants, donner un aspect plus épais, renforcer les ongles ou cheveux mous, et stimuler la croissance. Dans un mode de réalisation particulier, l'application n'est pas suivie d'un rinçage. Quel que soit le mode de réalisation, l'application peut s'effectuer sur tous types de phanères, notamment sur des cheveux naturels, des cheveux normaux, des cheveux abîmés, des cheveux secs, des cheveux colorés ou des cheveux cassants.

D'autres avantages pourront encore apparaître à l'homme du métier à la lecture des exemples ci-dessous, donnés à titre illustratif.

### Brève description des figures

[Fig. 1] représente l'intensité de la filaggrine par plan (couches du micro-épiderme) pour le contrôle (trait plein), le micro-épiderme (ME) traité avec un extrait de phacélie à 10⁻⁴% (tirets) et le ME traité avec un extrait de phacélie à 10⁻³% (pointillés).
[Fig. 2] représente l'expression de filaggrine totale (en pourcentage par rapport au témoin) pour le contrôle, le micro-épiderme (ME) traité avec un extrait de phacélie à 10⁻⁴% et le ME traité avec un extrait de phacélie à 10⁻³%.

### EXEMPLES

### Exemple 1 : Préparation de l'extrait de phacélie

Un extrait utilisé dans l'invention est préparé selon le procédé comprenant les étapes suivantes :
- mise en contact des parties aériennes fleuries de phacélie, broyées, avec un solvant hydroéthanolique, c'est-à-dire avec un mélange eau/ éthanol ayant un rapport massique 70/30. La quantité de plante utilisée par rapport au solvant est de 7% en poids par rapport au poids total de plantes et de solvant,
- extraction pendant une durée de l'ordre de 1H30 min à 50°C,
- filtration pour la séparation du résidu sec et du jus d'extraction et jusqu'à une filtration stérilisante,
- concentration sous vide de l'extrait filtré,
- ajout de glycérine pour finaliser l'extrait,
- éventuellement une pasteurisation et/ou filtration stérilisante de l'extrait obtenu dans les étapes antérieures.

### Exemple 2 : Préparation de l'extrait de phacélie

Un extrait utilisé dans l'invention est préparé selon le procédé comprenant les étapes suivantes :
- mise en contact des graines de phacélie avec du CO2 supercritique à 250 bars à 60°C,
- retour à pression atmosphérique de l'extrait final.

### Exemple 3 : Préparation de l'extrait de phacélie

Un extrait est préparé selon le procédé comprenant les étapes suivantes :
- mise en contact des parties aériennes fleuries de phacélie, broyées, en contact avec une huile de tournesol. La quantité de plante utilisée par rapport au solvant est de 7% en poids par rapport au poids total de plantes et de solvant,
- extraction avec des ultra-sons,
- filtration pour la séparation du résidu sec et du jus d'extraction

### Exemple 4 : Effet de l'extrait de phacélie sur les mécanismes de l'angiogenèse

L'angiogenèse est un terme biologique qui regroupe de nombreux processus reliés au développement du réseau sanguin (artères, vaisseaux, capillaires...). Dans le derme, les capillaires sanguins se forment par l'agencement tridimensionnel des cellules endothéliales. Ces cellules sont particulièrement jointives et développent de nombreuses ramifications pour assurer le réseau qui irrigue, alimente et oxygène tout le tissu cutané. Favoriser ces interactions est un mécanisme angiogène.

Les zones de bulbe et les matrices unguéales étant particulièrement vascularisées, une bonne angiogenèse à leur niveau permet d'assurer une bonne croissance des ongles et des cheveux/cils/sourcils.

Dans cette expérience, des cellules endothéliales transduites (GFP-HUVEC) pour mise en évidence du cytoplasme par marquage fluorescent, sont cultivées avec des fibroblastes humains normaux dans un mélange de milieu Endothelial Cell Growth 2 supplémenté avec hEGF, hbFGF, R3 IGF, hydrocortisone, vitamine C, et de milieu DMEM supplémenté avec 2 mM L-glutamine, 50 U/ml pénicilline, 50µg/ml streptomycine et 1% de sérum de veau fœtal au ratio 50/50. Les cellules en co-culture sont traitées, ou non, avec le VEGF à 100 ng/ml (Sigma, réf. V7259), ou encore avec l'extrait de phacélie à 10-3% et 10-4% (p/v). Les traitements ont lieu à J1 et J4 en milieu faible en sérum. Les points de branchement sont comptés pour évaluer les capacités des cellules endothéliales à former leur réseau en temps réel grade à Incucyte SIII. Les pourcentages sont calculés en fonction du contrôle milieu. Le VEGF (référence positive) valide les manipulations.

**[Table 1]**

| Actif | Points de branchements |
|---|---|
| Phacélie 10⁻³% | +55% |
| Phacélie 10⁻⁴% | +23% |
| VEGF 50ng/mL | +473% |

L'extrait de phacélle présente un effet stimulant sur le processus d'angiogenèse par augmentation du nombre de branchements inter-cellulaires. A savoir, pour la concentration la plus faible, +55% de points branchements entre cellules, +23»% à la plus forte.

L'extrait de phacélie permet donc stimuler un paramètre associé à l'angiogenèse, et permet donc de mieux nourrir, hydrater et oxygéner les zones de bulbe et les matrices unguéales pour des effets esthétiques visibles sur les ongles et les cheveux/cils/sourcils.

### Exemple 5 : Effet de l'extrait de phacélie sur les mécanismes de différenciation kératinocytaire.

La différenciation des kératinocytes pilaires est le processus qui permet l'édification de cellules fusiformes fortement solidarisées, chargées de fibrilles presque exclusivement constituées par l'assemblage de molécules fibreuses de kératine. Ces cellules perdent leurs organites, leur noyau dégénère et elles finissent par mourir. Elles sont responsables des propriétés physiques de la partie apparente de la tige du cheveu.

Ce mécanisme est à rapprocher de celui impliquant la différenciation des kératinocytes lors de l'édification de la fonction barrière à la surface de l'épiderme constituée de kératinocytes cornifiés. Durant ce processus, ils perdent leur capacité de prolifération et évoluent biochimiquement en exprimant des kératines particulières ou des marqueurs plus tardifs comme la filaggrine. Cette différenciation est accessible en culture monocouche mais reste imparfaite, c'est pourquoi l'utilisation de modèle tridimensionnel est plus pertinent scientifiquement pour mesurer un effet sur ce processus.

Les tests ont été réalisés sur des micro-épidermes Episcreen^{™} (société Cytoo) reconstruits à partir de kératinocytes humains néonataux en P6, ensemencés sur plaques coatées au collagène I.

Des études préliminaires sont réalisées afin de pouvoir valider le modèle et l'essai, utilisant 2 témoins positifs : la trichostatine A (TSA) et l'acide all-trans rétinoïque (ATRA) ; suivant 3 paramètres : la structure des micro-épidermes, le nombre de noyaux par micro-épiderme, les expressions des marqueurs Ki-67 et filaggrine et 2 points de cinétique, J2 et J4.

Les micro-épidermes différencient normalement en condition contrôle, atteignant le niveau d'expression attendu de filaggrine.

ATRA stimule la prolifération des kératinocytes induisant une augmentation significative du nombre de noyaux dans la couche basale. Comme les micro-épidermes se différencient fortement et rapidement, le pourcentage de Ki-67 exprimé par les cellules est faible et variable pour la condition contrôle, mais tend à augmenter après traitement avec le témoin ATRA.

TSA stimule la différenciation des micro-épidermes, ce qui se traduit par une augmentation significative de l'expression de la filaggrine.

Le traitement avec l'extrait de phacélie 10⁻³% et 10⁻⁴% (p/v) ne présente pas d'impact majeur sur le nombre de noyaux comptés par micro-épiderme.

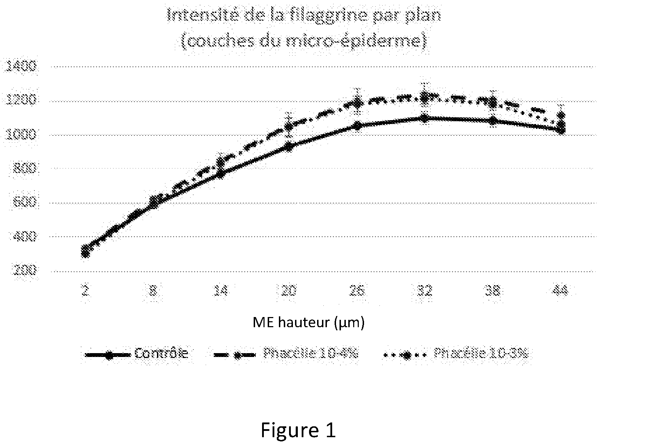

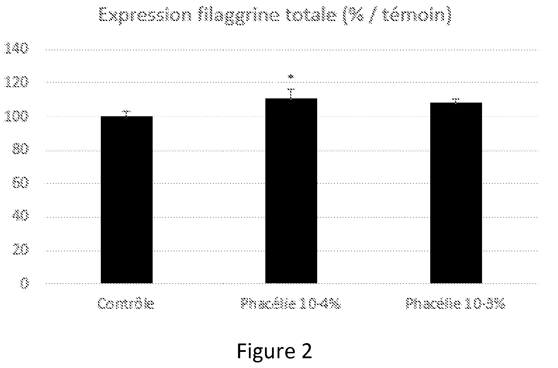

Globalement, les expressions de Ki-67 sont très faibles et peu variables. L'intensité de la filaggrine a été mesurée sur 8 plans z (voir Figure 1) et normalisée à la condition de contrôle (voir Figure 2).

L'extrait de phacélie présente des propriétés stimulant la différenciation des kératinocytes. En effet, l'expression de la filaggrine par les cellules est augmentée en présence de cet extrait, et notamment de manière significative (+11%) à la concentration 10-4% (p/v).

Sur les différents plans du micro-épiderme, l'effet de l'extrait de phacélie est le plus important entre 14µm et 38µm d'épaisseur, soit les couches les plus hautes, donc les plus différenciées.

L'extrait de phacélie stimule donc l'expression de la filaggrine, la différenciation kératinocytaire et de ce fait la kératinisation des phanères.

### Exemple 6 : Formulation d'un extrait de phacélie dans un sérum concentré pour soin végétal capillaire

**[Table 2]**

| Composants (nom INCI) | Pourcentage (% en poids) |
|---|---|
| AQUA | 55,575 |
| GELLAN GUM | 0,150 |
| SODIUM CHLORIDE | 0,075 |
| SODIUM BENZOATE | 0,500 |
| POTASSIUM SORBATE | 0,200 |
| SALICYLIC ACID | 0,150 |
| BETAINE | 2,000 |
| LACTIC ACID & AQUA | 0,150 |
| PANTHENOL | 0,200 |
| ALOE BARBADENSIS LEAF JUICE | 40,000 |
| Extrait de PHACELIE | 1,000 |

### Exemple 7 : Formulation d'un extrait d'Agave dans un sérum concentré pour soin végétal capillaire

**[Table 3]**

| Composants (nom INCI) | Pourcentage (% en poids) |
|---|---|
| AQUA | 54,575 |
| GELLAN GUM | 0,150 |
| SODIUM CHLORIDE | 0,075 |
| SODIUM BENZOATE | 0,500 |
| POTASSIUM SORBATE | 0,200 |
| SALICYLIC ACID | 0,150 |
| BETAINE | 2,000 |
| LACTIC ACID & AQUA | 0,150 |
| PANTHENOL | 0,200 |
| ALOE BARBADENSIS LEAF JUICE | 40,000 |
| Extrait de PHACELIE | 1,000 |

### Exemple 8 : Formulation d'un extrait d'Agave dans un shampooing

**[Table 4]**

| **Composants (nom INCI)** | **Pourcentage (% en poids)** |
|---|---|
| AQUA | 76,660 |
| GUAR HYDROXYPROPYLTRIMONIUM CHLORIDE | 0,150 |
| CITRIC ACID | 0,090 |
| SODIUM BENZOATE | 0,500 |
| ZINC GLUCONATE | 0,500 |
| SALICYLIC ACID | 0,050 |
| Extrait de PHACELIE | 0,125 |
| COCO-GLUCOSIDE & GLYCERYL OLEATE & AQUA | 3,000 |
| DECYL GLUCOSIDE & AQUA | 3,000 |
| AMMONIUM LAURYL SULFATE & AQUA | 10,400 |
| COCAMIDOPROPYL BETAINE & AQUA | 4,000 |
| PARFUM | 0,400 |
| URTICA DIOICA EXTRACT & AQUA & GLYCERIN | 1,000 |

### Exemple 9 : Formulation d'un extrait d'Agave dans un soin nutrition pour les cheveux sous forme d'après-shampooing

**[Table 5]**

| **Composants (nom INCI)** | **Pourcentage (% en poids)** |
|---|---|
| AQUA | 87,967 |
| Extrait de PHACELIE | 0,250 |
| FRUCTOOLIGOSACCHARIDES | 0,250 |
| PANTHENOL | 0,100 |
| SODIUM BENZOATE | 0,350 |
| BEHENTRIMONIUM CHLORIDE & ISOPROPYL ALCOHOL | 3,000 |
| STEARYL ALCOHOL | 3,500 |
| CETYL ALCOHOL | 2,000 |
| PERSEA GRATISSIMA OIL | 1,000 |
| COCOS NUCIFERA OIL | 1,000 |
| PARFUM | 0,400 |
| OLEA EUROPAEA FRUIT OIL & BETA-CAROTENE | 0,003 |
| CITRIC ACID | 0,180 |

## Revendications

1. Utilisation cosmétique non thérapeutique d'au moins un extrait hydrophile de phacélie appartenant à l'espèce *Phacelia tanacetifolia,* choisi parmi un extrait de feuille, de tige, de fleur, de graine, ou l'un de leurs mélanges pour le soin, le nettoyage et/ou le maquillage des phanères.

2. Utilisation selon la revendication 1, dans laquelle ledit extrait est un extrait des parties aériennes fleuries.

3. Utilisation selon la revendication 1 ou 2, dans laquelle ledit au moins un extrait hydrophile est choisi parmi un extrait alcoolique, un extrait aqueux, un extrait hydro-alcoolique, un extrait glycolique, et un extrait obtenu sous haute pression avec des solvants à l'état super ou subcritque, par entraînement à la vapeur d'eau.

4. Utilisation selon la revendication 3, dans laquelle ledit extrait est un extrait hydro-alcoolique.

5. Utilisation selon l'une quelconque des revendications précédentes, ledit au moins un extrait étant dans une composition cosmétique comprenant de 0,01 et 10% en poids dudit extrait par rapport au poids total de la composition et au moins un véhicule cosmétiquement acceptable.

6. Utilisation selon la revendication 5, ladite composition étant sous une forme choisie parmi un onguent, une crème, une huile, un shampooing, un après-shampooing, un produit coiffant, un vernis à ongles, un mascara, une solution, un gel, un baume, un beurre, une lotion, une suspension et une émulsion.

## Patentansprüche

1. Nicht-therapeutische kosmetische Verwendung mindestens eines hydrophilen Extrakts aus Phacelia der Art *Phacelia tanacetifolia,* ausgewählt aus einem Extrakt aus Blättern, Stängeln, Blüten, Samen oder einer Mischung davon, zur Pflege, Reinigung und/oder zur dekorativen kosmetischen Behandlung der Hautanhangsgebilde.

2. Verwendung gemäß Anspruch 1, wobei der Extrakt ein Extrakt aus den blühenden oberirdischen Teilen ist.

3. Verwendung gemäß Anspruch 1 oder 2, wobei mindestens ein hydrophiler Extrakt ausgewählt ist aus einem alkoholischen Extrakt, einem wässrigen Extrakt, einem hydroalkoholischen Extrakt, einem Glykolextrakt und einem unter Hochdruck mit überkritischen oder unterkritischen Lösungsmitteln, insbesondere mittels Wasserdampfbehandlung, gewonnenen Extraktt.

4. Verwendung gemäß Anspruch 3, wobei der Extrakt ein hydroalkoholischer Extrakt ist.

5. Verwendung gemäß einem der vorstehenden Ansprüche, wobei der mindestens eine Extrakt in einer kosmetischen Zusammensetzung enthalten ist, die 0,01 bis 10 Gew.-% des Extrakts, bezogen auf das Gesamtgewicht der Zusammensetzung, und mindestens einen kosmetisch akzeptablen Träger enthält.

6. Verwendung gemäß Anspruch 5, wobei die Zusammensetzung in einer Form vorliegt, die aus einer Salbe, einer Creme, einem Öl, einem Shampoo, einem Conditioner, einem Styling-Produkt, einem Nagellack, einer Wimperntusche, einer Lösung, einem Gel, einem Balsam, einer Butter, einer Lotion, einer Suspension und einer Emulsion ausgewählt ist.

## Claims

1. Non-therapeutic cosmetic use of at least one hydrophilic extract of phacelia belonging to the species *Phacelia tanacetifolia,* chosen from among an extract of the leaf, stem, flower, seed, or a mixture thereof, for the care, cleansing and/or make-up of the skin appendages.

2. Use according to claim 1, wherein said extract is an extract from the flowering aerial parts.

3. Use according to claim 1 or 2, wherein said at least one hydrophilic extract is selected from an alcoholic extract, an aqueous extract, a hydroalcoholic extract, a glycolic extract, and an extract obtained under high pressure with supercritical or subcritical solvents by steam distillation.

4. Use according to claim 3, wherein said extract is a hydroalcoholic extract.

5. Use according to any one of the preceding claims, said at least one extract being in a cosmetic composition comprising from 0.01 to 10% by weight of said extract relative to the total weight of the composition and at least one cosmetically acceptable vehicle.

6. Use according to claim 5, wherein said composition is in a form selected from an ointment, a cream, an oil, a shampoo, a conditioner, a styling product, a nail polish, a mascara, a solution, a gel, a balm, a butter, a lotion, a suspension and an emulsion.
